# EUROPEAN PATENT SPECIFICATION

(11) **EP 4 331 626 B1**
(45) Date of publication and mention of the grant of the patent: **30.07.2025**
(21) Application number: 22193627.1
(22) Date of filing: 02.09.2022
(51) Int. Cl.: A61L 2/07, A61L 2/24, B01J 3/04, B01D 19/00

(54) **A METHOD OF CONTROLLING A STEAM STERILIZER SYSTEM**
VERFAHREN ZUM STEUERN EINES DAMPFSTERILISIERUNGSSYSTEMS
PROCÉDÉ DE COMMANDE D'UN SYSTÈME DE STÉRILISATEUR À VAPEUR

(43) Date of publication of application: 06.03.2024
(73) Proprietor: Getinge Sterilization AB, 305 05 Getinge (SE)
(72) Inventor: STORBERG, Bengt, 311 70 Falkenberg (SE); HARAMBASIC, Elvir, 305 72 Steninge (SE)
(74) Representative: Zacco Sweden AB

(56) References cited:
- JP-A- 2002 081 611
- US-A1- 2014 334 977
- US-A1- 2020 038 778

## Description

### TECHNICAL FIELD

The present invention relates to a method of controlling a steam sterilizer system. The present invention also relates to a control unit configured to carry out the steps of such a method. The present invention further relates to a steam sterilizer system comprising such a control unit. Steam sterilizer systems can be used for processing used medical goods in a hospital central sterile services department (CSSD), for example.

### BACKGROUND ART

Inactivation of microorganisms by means of a steam sterilization process involves the presence of steam (moist heat) and high temperatures over a certain time period. During such a steam sterilization process, the material to be sterilized must be in contact with the steam and its condensate for said time period. However, there is a challenge in such steam sterilization processes because non-condensable gases (NCGs) may accumulate, and may insulate and prevent full steam penetration into for example lumens, such as in long hoses, textile packages and crevices on objects, and may thus impair the ability for inactivation of microorganisms in such locations. It is desirable to remove non-condensable gases (NCGs) to achieve an efficient steam sterilization process.

The NCGs are mainly dissolved O₂, N₂, and CO₂ that are absorbed from the atmosphere in lakes, rivers and wells. There may be a seasonal variation of NCG levels due to different temperatures and increased stirring of lakes during spring and fall when temperatures change.

A common method for removing NCGs from water for sterilization processes is hot well degassing. This involves using a heated tank where the feed water is kept at an elevated temperature. The saturation of dissolved gas decreases as the temperature rises. However, the degassing works poorly if sufficient time is not given for the water to heat up and the gases to escape, or if the temperature is too low. Furthermore, the temperature needs to be kept well below the boiling point in order to avoid damaging the pump or even for the water to be able to be pumped at all.

Examples are disclosed by : US2014334977 , US2020038778 and JP2002081611.

In view of the above it should be understood that there is still room for improvement when it comes to degassing the water that is to be used in a steam sterilization process.

### SUMMARY OF THE INVENTION

An object of the present invention is to present a method and system which at least partly alleviates the drawbacks of the prior art. This and other objects, which will become apparent in the following, are accomplished by a method as defined in claim 1. Some non-limiting exemplary embodiments are presented in the dependent claims.

The applicants have determined that water should be passed through a degassing filter and then recirculated through the degassing filter, thereby enabling a considerable reduction of the NCG content in the water before providing it to the boiler.

Thus, according to at least one aspect of the present inventive concept, there is provided a method of controlling a steam sterilizer system comprising a boiler in which liquid water is turned into steam, the method comprising:
- pumping water from a container and passing the pumped water through a degassing filter located upstream of the boiler in order to remove non-condensable gases (NCGs) dissolved in the water,
- subsequently to passing the water through the degassing filter, recirculating the water through the degassing filter for additional removal of NCGs, while preventing water from flowing from the degassing filter to the boiler, and
- subsequently to recirculating the water through the degassing filter, passing at least a portion of the water to the boiler.

By recirculating the water through the degassing filter, the NCG content will gradually be reduced, thereby enabling water having a low or substantially no NCG-content to be passed to the boiler for generating improved steam for the steam sterilizer.

Suitably, the steam sterilizer system may comprise a valve located downstream of the degassing filter and upstream of the boiler. In at least some exemplary embodiments of the method, said step of passing at least a portion of the water to the boiler may comprise opening said valve. In other words, the valve being located in a feed passage between the filter and the boiler may be controlled to be closed while recirculating the water through the degassing filter in order to prevent water from prematurely flowing to the boiler (i.e. before a sufficient amount of NCGs has been removed). The opening and closing of the valve may be controlled strategically by means of a control unit of the steam sterilizer system. Such a control unit provides additional advantages, and will be discussed in more detail later in this disclosure.

Said valve in a feed passage between the filter and the boiler may, for convenience, be referred to as a first valve. The steam sterilizer system may also have other valves. For instance, in order to recirculate the water, there is suitably provided a recirculation passage which extends to the container from a point downstream of the degassing filter. In such a recirculation passage, there may be may be provided a valve which, for convenience, may be referred to as a second valve. When the first valve is closed, the second valve may be controlled to be open to allow for recirculation of the water through the degassing filter. When the time comes to open the first valve to allow at least a portion of the water to be passed to the boiler, then the second valve may be closed. It should, however, be understood, that closing of the second valve is not necessary. Indeed, some recirculation may be allowed simultaneously with passing water to the boiler. For instance, instead of a controllable second valve the recirculation passage may be provided with a small orifice which is always open for recirculation. From the above it should be understood that, according to at least one exemplary embodiment of the method, the method may comprise, simultaneously with the step of passing at least a portion of the water to the boiler, continuing recirculating a minor part of the water that has passed through the degassing filter.

It should be understood that the general idea of recirculating the water through the degassing filter while preventing the water from flowing from the degassing filter to the boiler, may be implemented in various ways. For instance, in some exemplary embodiments, a relatively short recirculation loop may conceivable, with or without a separate pump, in which case water that has exited the degassing filter may be returned to the filter without passing through the container. In other words, in at least some exemplary embodiments, the recirculation of the water may bypass the container. However, as has already been indicated above, in at least some exemplary embodiments the recirculation may advantageously be performed via the container. Thus, according to at least some exemplary embodiments, the step of recirculating the water through the degassing filter may comprise returning the water to the container and then pumping it to the degassing filter again.

According to at least one exemplary embodiment, said step of recirculating the water through the degassing filter comprises:
- recirculating water through the filter so that the total volume that is passed through the degassing filter before said at least a portion of the water is passed to the boiler is at least double the volume of water that was initially present in the container before performing said step of pumping water from the container.

Thus, the water is suitably recirculated "multiple times", so that all of the water in the container has passed through the degassing filter, on average, at least twice. The more times the water is recirculated, the higher amount of NCGs will be removed from the water. The actual control of the recirculation may, of course, be volume-based, e.g. using a flow meter. However, suitably, the recirculation may be time-based, i.e. recirculating for a certain time period, which may correspond to a certain volume of water having been passed through the degassing filter.

According to at least one exemplary embodiment, the step of recirculating the water through the degassing filter is continued until determining that either (i) the water has been circulated through the degassing filter for at least a minimum time period, T1, or (ii) the water has completed at least a minimum number of cycles through the degassing filter.

The minimum time period T1 may, for example, be determined empirically, or through a look-up table, or be calculated based on known or estimated NCG content, flow rate, volume, etc. The minimum time period T1 is suitably selected such that a satisfactory reduction of NCGs in the circulating water is achieved before water is allowed to pass to the boiler. If time allows, and there is no urgent or pending request to supply the boiler with water, then recirculation may suitably continue for a longer time period than said minimum time period T1, in order to even further reduce the NCG content. In case the control of the recirculation is not time-based, and instead a minimum number of cycles is to be completed, then such control may be implemented by using a flow meter to measure the volume of water that has passed through the degassing filter. Thus, one cycle may suitably correspond to passing a volume of water through the degassing filter which corresponds to the volume that was initially present in the container before starting to pump water from the container. In some embodiments, the volume of water passing through the degassing filter before the water is passed to the boiler is at least double the volume of water initially present in the container, or at least double the volume of water initially present in a degassing circulation loop.

In addition to the above-mentioned time-based and cycle-based alternatives for controlling the duration of the recirculation, another conceivable alternative is a pressure-based control. Such a pressure-based control may suitably be implemented in connection with a vacuum tank being used for drawing NCGs from the degassing filter. This will be explained in more detail below.

According to at least one exemplary embodiment, the method comprises applying, by means of a vacuum tank, a vacuum to the degassing filter to draw NCGs from water passing through the degassing filter.

Applying a vacuum to the degassing filter improves the gas removal efficiency of the degassing filter. The vacuum tank may, for example, be connected to a central vacuum arrangement of the sterilizer system or may instead be connected to a separate ejector or an additional vacuum pump.

As indicated above, a pressure-based control of the recirculation duration may suitably be implemented, Thus, according to at least one exemplary embodiment, the step of recirculating the water through the degassing filter is continued until determining that there is a pressure rate of change below a predefined threshold in the vacuum tank.

Since a vacuum tank will normally be provided with a pressure sensor, use of such a pressure sensor may be convenient for monitoring the change in gas content during recirculation of the water. In particular, it should be understood that, initially, when you start the degassing process, the water will have a high NCG content, and a rather steep pressure rise in the tank is expected at the beginning of the degassing (recirculation) process. However when the water is fully degassed, then continued recirculation should not result in a pressure rise. Thus, by monitoring the pressure rate of change, the control unit may determine when the recirculating water is sufficiently degassed.

Although it may be convenient to measure the pressure rate of change in the vacuum tank as discussed above, it should be understood that such pressure rate of change may instead, or additionally, be measured at other locations. Indeed, regardless of using a vacuum tank or some other device for creating a vacuum, the pressure rate of change may be measured in any suitable closable volume which can be arranged in gaseous communication with the degassing filter. Thus, in a general sense, and according to at least one exemplary embodiment, the step of recirculating the water through the degassing filter is continued until determining that there is a pressure rate of change below a predefined threshold in a closable volume in fluid communication with the degassing filter.

A pressure sensor may be provided in any such volume, be it the previously discussed vacuum tank or another volume in fluid communication with the degassing filter. By fluidly connecting the volume to a dry side of the filter and then closing the volume during measurement, a pressure value may be obtained from the pressure sensor. Gas molecules passing through the degassing filter will raise the pressure in the volume. If there is already a pressure in the volume (e.g. no or poor vacuum) then there will be no or little transport of gas molecules.

Although the above-mentioned time-based, cycle-based and pressure-based control strategies have been presented as different conceivable alternatives, it should be understood that combinations thereof are also conceivable. For instance, another possibility is to control the recirculation based on a combination of the pressure rate of change and the minimum time period T1, which will result in an even more robust control strategy.

Some additional discussion relating to the use of a vacuum tank will now follow. In particular, various tests may be executed in connection with such a vacuum tank. One such test, may be a leak test, to make sure that the provided vacuum functions to satisfaction. Another such test, is a performance test.

Thus, according to at least one exemplary embodiment, the method comprises:
- subsequently to recirculating the water through the degassing filter but before passing said at least a portion of the water to the boiler, performing a vacuum leak test by:
- allowing the water to stabilize during a first duration of time, and subsequently
- determining during a second duration of time, the pressure in the vacuum tank, and subsequently
- determining that the leak test has been passed if any increase of pressure in the vacuum tank during the second duration of time is below a first predefined pressure change.

Additionally, according to at least one exemplary embodiment, the method comprises:
- upon determining that the leak test has been passed and subsequently to or while passing said at least a portion of the water to the boiler, performing a performance test by:
- supplying the container with fresh water not previously degassed, and subsequently
- recording a start pressure in the vacuum tank, and subsequently
- recirculating the supplied fresh water through the degassing filter, and subsequently
- recording a stop pressure in the vacuum tank, and subsequently
- determining that the degassing performance is satisfactory if the difference between the stop pressure and the start pressure is above a second predefined pressure change.

The above leak test and performance test are clearly beneficial for reducing the risk of incorrect control and degassing. The leak test and/or the performance test may form part of separate maintenance cycles, or may be integrated into the production cycles. Any leak will make degassing more difficult, requiring more vacuum support and may result in the above-mentioned pressure-based recirculation control to become ineffective. A lack of pressure rise in the vacuum tank during initial degassing of water that has not been degassed before, may be indicative of a faulty, worn or clogged filter that needs to be replaced.

As an alternative or a supplement to the above-discussed control strategies (time-based, volume-based and/or pressure-based), another control strategy may be to monitor the actual NCG content in the container. Thus, according to at least one exemplary embodiment, the method comprises:
- monitoring the content of NCGs in the container,
- proceeding from said step of recirculating the water through the degassing filter to said step of passing at least a portion of the water to the boiler when the content of NCGs has decreased to or below a predefined threshold value.

Thus, this is another way to avoid premature filling of water (with high NCG content) into the boiler. Monitoring the NCG content of the water in the container may, for example, be achieved by using an in situ Total Dissolved Gas (TDG) sensor between in the discharge passage from the container to the degassing filter. Suitably, such a TDG sensor may be arranged in the discharge passage between a circulation pump and the degassing filter. Another possibility is to use a single-gas sensor, e.g. an oxygen gas (O₂) sensor. Since a certain relationship between the quantities of O₂, N₂ and CO₂ can be expected in the fresh water, a sensor which only measures the quantity of one of the gases may therefore be sufficient for estimating/calculating the total NCG content.

As explained above, there may be various control strategies to reduce the risk of the water being passed prematurely to the boiler, in particular to reduce the risk of filling the boiler with water having too high NCG content. There may, however, be other considerations for determining when to allow water to enter the boiler. This is reflected in at least one exemplary embodiment, according to which the method comprises:
- monitoring the liquid water level in the boiler,
- proceeding from said step of recirculating the water through the degassing filter to said step of passing at least a portion of the water to the boiler when the liquid water level in the boiler has reached or fallen below a predefined water level.

Thus, by monitoring the liquid water level in the boiler the control unit may determine if there remains sufficient water for steam generation for an upcoming sterilization of articles to be sterilized in a sterilizer downstream of the boiler. The boiler may therefore suitably be provided with a level control system including level sensors for detecting when the level in the boiler is low, as a consequence of water therein having been converted into steam by means of for example heating elements.

According to at least one exemplary embodiment, the method comprises:
- determining that the water level in the boiler will within a determined time period, T2, reach or fall below said predefined water level, and
- preventing fresh water from being supplied to the container until said predefined water level in the boiler has been reached and degassed water from the container has been passed to the boiler to refill the boiler,
- subsequently to said step of preventing fresh water from being supplied, supplying fresh water to the container.

Supplying fresh water to the container results in an increased NCG content when mixed with any degassed water already present in the container. It is therefore advantageous to prevent fresh water from being supplied to the container if the boiler is to be filled in the near future.

The determined time period T2 may suitably be calculated based on current circumstances, and may vary from one occasion to another. If the determined time period T2 is short, which may be indicative of a re-filling of the boiler being required shortly, then it is advantageous to suspend supplying of fresh water to the container until after the re-filling of the boiler has been completed, and thus only use degassed water already present in the container. This may be particularly relevant if the container volume is significantly larger than the re-filling volume normally passed to the boiler, as the water in the container can then be used for several batches of re-filling and steam generation operations. Conversely, it should be understood that if the determined time period T2 is relatively long, then there may be sufficient time to fill up the container with fresh water and have it recirculated through the degassing filter to reduce the NCG content sufficiently before re-filling of the boiler is expected to be requested. For instance, if using the time-based control, if the time period T2 is longer than the sum of the time it takes to fill the container and the minimum time period T1 for recirculating the water through the degassing filter, then fresh water may suitably be supplied to the container. Naturally, the control unit may be configured to calculate, based on time periods T2 and T1 and the fresh water supply rate, how much fresh water is allowed to be supplied to the container (if a complete filling would take too long), such that the supplied amount of fresh water can be degassed in a timely manner within the time period T2.

It should be understood that supplying fresh water to the container may be made from a fresh water conduit directly to the container. However, another conceivable option is to add the fresh water upstream or downstream of the container in the degassing arrangement. For instance, the fresh water may be added to the above mentioned recirculation passage which interconnects a point downstream of the degassing filter with the container, or the fresh water may be added to a discharge passage which interconnects the outlet of the container with the degassing filter. If a pump is provided in the discharge passage than the fresh water may be added to the discharge passage upstream or downstream of the pump. Thus, it should be understood that the fresh water may be supplied to the container by letting the fresh water enter at any suitable location of the recirculation path of the water in the degassing arrangement where it will reach the container during degassing circulation.

Similarly to the above discussed timing of preventing fresh water from being supplied to the container, according to at least one exemplary embodiment, the method comprises:
- determining if the boiler will require added water within a predetermined time period, in particular based on at least one of a water level in the boiler and a process stage of the steam sterilizer system; and
- preventing fresh water from being supplied to the container if the boiler will require added water within the predetermined time period.

The predetermined time period may, for example, correspond to the previously discussed predetermined time period T2.

The idea of timing the supply of fresh water to the container is also reflected below. Thus, according to at least one exemplary embodiment, the method further comprises:
- preventing fresh water from being supplied to the container while water is recirculated through the degassing filter, and subsequently
- passing at least a portion of said water to the boiler, and subsequently
- supplying fresh water to the container.

If the container is filled with approximately the same volume of water that is later passed to the boiler, then the above steps may suitably be repeated for each new batch of water to be evaporated in the boiler.

According to at least one exemplary embodiment, the method comprises:
- monitoring the water level in the container, and
- controlling the supply of fresh water to the container based on the current water level in the container. In particular, controlling the supply of fresh water to the container based on whether the current water level in the container is expected to be sufficient for a next batch of water to be provided to the steam generator.

By monitoring the water level in the container, unnecessary dilution may be avoided. The control unit may suitably be configured to determine if the current water level is sufficient for another batch of steam generation in the boiler, and if that is the case, then supply of fresh water to the container may be prevented. In other words, it may be better to make use of the already degassed water available in the container before supplying fresh water to the container, as the fresh water would increase the NCG content in the container and require further circulation of the water through the degassing filter.

According to at least one exemplary embodiment, said step of controlling the supply of fresh water to the container based on the current water level in the container comprises:
- supplying fresh water to the container when the water level is below a predefined level.

As understood from the previous discussions, supplying water into the container when the water level is low is advantageous as it reduces a rise in dissolved gas level.

The prevention of providing fresh water to the container may suitably be achieved by means of a further valve, a container-filling valve, which can be opened and closed. Such a container-filling valve may be controlled by the control unit based on level sensor input (e.g. from one or more sensors monitoring the water level in the container). However, in at least some alternative exemplary embodiments, the container may be provided with a float valve instead of the container-filling valve and the level sensors.

According to at least one exemplary embodiment, the method further comprises at least one of:
- providing fresh water to the container in response to a determination that degassed water will not be provided to the boiler for at least a first time period, T3; and
- preventing providing fresh water to the container in response to a determination that water will need to be provided to the boiler within a second time period, T4.

In embodiments in which the method also takes uses the previously discussed time-based control and the predetermined time period T1, then said first time period T3 should normally be greater than the time period T1, since in addition to the recirculation time (T1), time will also elapse during the actual feeding of fresh water to the container. Said second time period T4 may, however, in at least some exemplary embodiments correspond to the previously discussed predetermined time period T2, i.e. a predicted time period for the water to fall to or fall below a predefined level in the boiler.

According to at least one exemplary embodiment, the method comprises:
- subsequently to said step of passing at least a portion of the water to the boiler, preventing water from passing to the boiler, and
- supplying fresh water to the container, and then
- reiterating said steps of pumping and recirculating water through the degassing filter while preventing water from flowing from the degassing filter to the boiler.

In other words, according to this exemplary embodiment, the control of supplying fresh water may advantageously be based on completed/finished filling of boiler, e.g. directly after (within a short time period after) filling the boiler.

In other exemplary embodiments, the container may be associated with a predefined low water level and a predefined high water level. In such embodiments, and when the NCG level is assumed to be low as a result of degassing, you will normally not add fresh water to the container when the current water level is between said predefined low and high water levels. Instead, the water already present in the container will be used for filling the boiler with degassed water when the boiler may require it. This may be done as long as there is no risk of running out of degassed water in the container. When the current water level has dropped to or below said predefined low water level, then fresh water may again be supplied to the container. This will raise the NCG content in the container, and a new degassing/circulation phase will be initiated. This is at least partly reflected in the following exemplary embodiment. According to at least one exemplary embodiment, the method comprises:
- subsequently to said step of passing at least a portion of the water to the boiler, operating the boiler to generate steam, and
- monitoring the water level in the container, and
- preventing providing fresh water to the container as long as the water level in the container is above or equal to a predefined water level, and
- subsequently to said step of operating the boiler to generate steam, and as long as the water level in the container is above or equal to said predefined water level, passing a further portion or portions of the water to the boiler and operate the boiler to generate steam.

Although it may be advantageous and convenient to have a clear threshold in the form of said predefined low water level for supplying fresh water to the container, the control unit may suitably be configured to handle other scenarios as well. For instance, the control unit may suitably determine that water may be supplied even though the current water level has not dropped to said predefined low water level, i.e. the current water level is still between the predefined low and high water levels. This will, however, be performed proactively if the control unit has determined that there is sufficient time for degassing supplied fresh water before water needs to be passed to the boiler.

According to at least another aspect of the invention, there is provided a control unit configured to carry out the steps of any of the above described methods (including any exemplary embodiment thereof) method according to any preceding claim, the control unit being configured to:
- control a pump of the steam sterilizer system, thereby controlling the pumping of water from the container,
- controlling a first valve of the steam sterilizer system to selectively prevent or enable water to be passed from the degassing filter to the boiler, and
- optionally, controlling a further valve of the steam sterilizer system to selectively prevent or enable fresh water to be supplied to the container.

The advantages provided by said control unit are largely analogous to the advantages provided by the previously disclosed method (including any exemplary embodiment thereof).

The control unit may include a microprocessor, microcontroller, programmable digital signal processor or another programmable device. The control unit may also, or instead, include an application specific integrated circuit, a programmable gate array or programmable array logic, a programmable logic device, or a digital signal processor. Where it includes a programmable device such as the microprocessor, microcontroller or programmable digital signal processor mentioned above, the processor may further include computer executable code that controls operation of the programmable device. The control unit is preferably configured for use with steam sterilizer systems, and provided with electronic instructions to perform and control any of the methods described herein.

According to at least yet another aspect of the present inventive concept there is provided a steam sterilizer system comprising the control unit according to the above-presented aspect (including any exemplary embodiment thereof), and further comprising a boiler and a sterilization chamber.

The advantages provided by said steam sterilizer system are largely analogous to the advantages provided by the previously disclosed control unit and the previously disclosed method (including any exemplary embodiments thereof).

In the following some additional aspects of the disclosure will be presented.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated. Further features of, and advantages with, the present invention will become apparent when studying the appended claims and the following description. The skilled person realizes that different features of the present invention may be combined to create embodiments other than those described in the following, without departing from the scope of the claims.

### BRIEF DESCRIPTION OF THE DRAWINGS

Fig. 1 is a schematic view of a steam sterilizer system according to at least one exemplary embodiment of the present invention.
Fig. 2 discloses schematically the steps of a method according to at least one exemplary embodiment of the present invention.
Fig. 3 is a schematic diagram of a steam sterilizer which could be used with the disclosed system.

### DETAILED DESCRIPTION

The present invention will now be described more fully hereinafter with reference to the accompanying drawings, in which certain aspects of the system are shown. The system may, however, be embodied in many different forms and should not be construed as limited to the embodiments set forth herein; rather, the embodiments are provided by way of example so that this invention will be thorough and complete, and will fully convey the scope of the system to those skilled in the art. Accordingly, it is to be understood that the present invention is not limited to the embodiments described herein and illustrated in the drawings; rather, the skilled person will recognize that many changes and modifications may be made within the scope of the appended claims. Like reference numerals refer to like elements throughout the description.

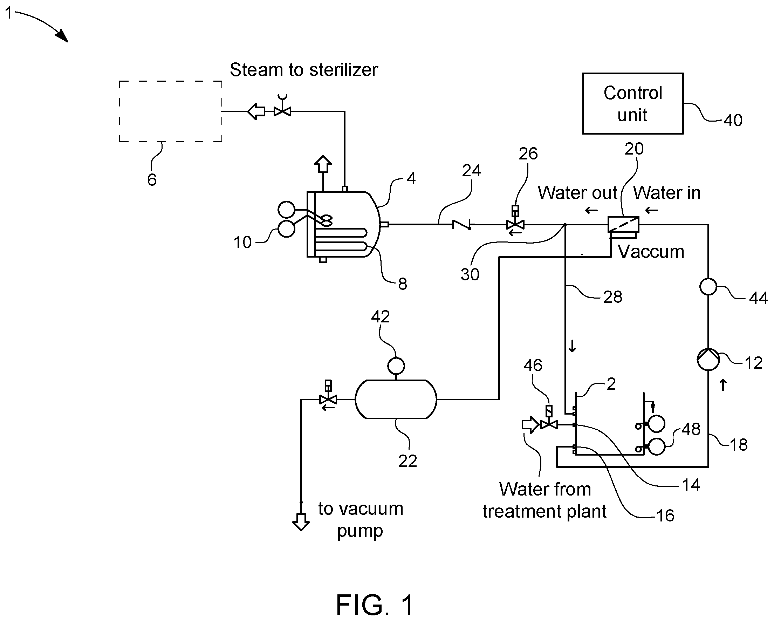

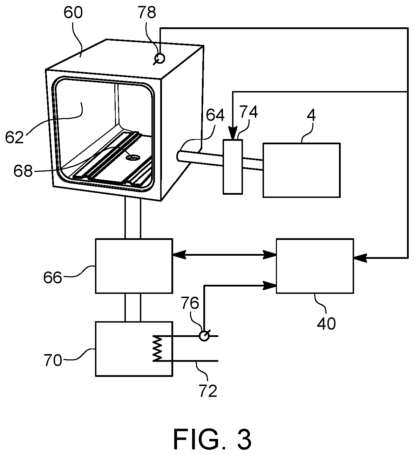

Fig. 1 is a schematic view of a steam sterilizer system 1 according to at least one exemplary embodiment. On a general level, the steam sterilizer system 1 comprises a container 2, a boiler 4 and a steam sterilizer 6. Water from the container 2 can be delivered to the boiler 4. The boiler 4 turns the delivered water into steam. The steam is then passed from the boiler 4 to the steam sterilizer 6. The steam sterilizer 6 is schematically depicted by a dashed rectangle and it should be understood that the present approach is not limited to the use of any particular type of steam sterilizer, but may be implemented with any suitable steam sterilizer having a sterilizing chamber in which articles, such as medical goods, to be sterilized can be subjected to steam generated by the boiler 4. An example of a steam sterilizer which could be used will be discussed later in connection with Fig. 3.

Continuing with Fig. 1, the boiler 4 may be provided with any suitable means for turning water into steam. For instance, the boiler 4 may be provided with one or more heating elements 8 within the boiler 4. Furthermore, the boiler 4 may be provided with one or more level switches 10 for interrupting the filling of water into the boiler 4.

The water may be fed to the boiler 4 from the container 2 by operating a pump 12 in order to pump water from the container 2. The container 2 may have an inlet 14 for supplying fresh water into the container 2 and an outlet 16 for discharging water from the container 2. The outlet 16 of the container 2 opens into a discharge passage 18 (conduit) which could be a pipe, tube, hose, or similar structure. The pump 12 is suitably provided in said discharge passage 18. As explained previously in this disclosure, if the water that is fed to the boiler 4 contains non-condensable gases (NCGs), these will together with the steam generated by the boiler 4 be passed on to the sterilizing chamber of the steam sterilizer 6. The presence of NCGs lowers the chances of achieving full steam penetration to all parts of the articles that are to be sterilized for the entire duration of a sterilization cycle. It is therefore desirable to remove most of the NCGs from the water before water is fed into the boiler 4. To achieve this effect, the steam sterilizer system 1 comprises a degassing filter 20.

The discharge passages 18 extends from the outlet 16 of the container 2 to the degassing filter 20. The degassing filter 20 may be of standard type used for vacuum or sweep gas degassing in various technical fields where water processing is performed. The degassing filter 20 may have a shell side, where the water passes along a hydrophobic membrane, and a lumen side (dry side), which is the other side of the membrane. The lumen side may be connected to a vacuum tank 22 held at low pressure. The degassing filter 20 works by striving for equilibrium of the gases on both sides of the membrane and thus moves gas dissolved in the water through the membrane to the vacuum side, where the gas is removed due to the connection to the vacuum tank 22. The water, however, does not pass through the membrane of the degassing filter 20.

A vacuum tank 22 may be use to maintain a vacuum, which may be created by means of a vacuum pump, which may be part of a dedicated vacuum system or shared vacuum system (e.g. shared with the steam sterilizer 6). Alternatively, the vacuum may be provided by a vacuum pump or system without a vacuum tank 22.

The water that exits the degassing filter 20 may continue downstream of the degassing filter 20 along a feed passage 24 for feeding water into the boiler 4. A first valve 26 may be provided in the feed passage 24 for controlling the flow of water through the feed passage 24. In particular, the first 26 valve may be controlled to a closed state in order to prevent water from passing to the boiler 4 from the degassing filter 20 when the pump 4 is pumping the water from the container.

A recirculation passage 28 extends from a point 30 of the feed passage 24 upstream of the first valve 26, but down of the degassing filter 20. The recirculation passage 28 is used for recirculating water that has passed through the degassing filter 20, back to the container 2, so that the same water may be passed through the degassing filter 20 multiple times before allowing at least a portion of the circulated water to be fed to the boiler 4. The container 2, the discharge passage 18 and the recirculation passage 28 can thus be regarded as forming part of a circulation loop.

The steam sterilizer system 1 also comprises a control unit 40 which is configured to communicate with various components of the steam sterilizer system 1, and is also configured to control the operation of certain components and to receive input signals from various components. The control unit 40 may be configured to communicate, send/receive instructions, etc. wirelessly or by wire. As such the control unit 40 is configured to perform the steps of the methods disclosed herein. The control unit 40 may be inside the same physical housing as the steam sterilizer 6, but in principal, it can also be at other locations. The control unit can include electronics, processors, memory, electronic instructions, and other elements that are useful to control any of the systems and methods described in this disclosure.

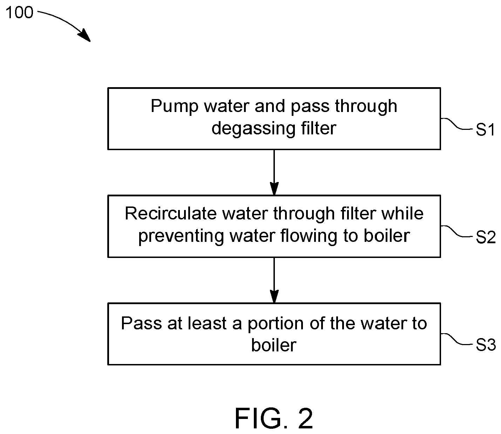

Fig. 2 discloses schematically a method 100 according to at least one exemplary embodiment of the present inventive concept. In particular, Fig. 2 discloses a method 100 of controlling a steam sterilizer system comprising a boiler in which liquid water is turned into steam. The steam sterilizer system may, for instance, be the steam sterilizer system 1 presented in Fig. 1 or a different one.

The method 100 comprises:
- in a first step S1, pumping water from a container and passing the pumped water through a degassing filter located upstream of the boiler in order to remove non-condensable gases (NCGs) dissolved in the water,
- in a step S2, subsequently to passing the water through the degassing filter, recirculating the water through the degassing filter for additional removal of NCGs, while preventing water from flowing from the degassing filter to the boiler, and
- in a step S3, subsequently to recirculating the water through the degassing filter, passing at least a portion of the water to the boiler.

If said method 100 is to be implemented for the steam sterilizing system 1 of Fig. 1, the control unit 40 may suitably be configured to control the pump 12 so that water is pumped from the container 2 through the outlet 16, along the discharge passage 18 and through the degassing filter 20, where NCGs dissolved in the water would be removed as water passes through the degassing filter 20. Thereby, the NCG content of the water becomes reduced. The control unit 40 may also control the first valve 26 to be actuated or maintained in a closed state, thereby closing off the feed passage 24, and thereby preventing water from flowing from the degassing filter 20 to the boiler 4. Instead water will be guided by the recirculation passage 28 back to the container 2 in order to recirculate the water through the degassing filter 20 for additional removal of NCGs. When sufficient degassing has been achieved, then the control unit 40 may control the first valve 26 to become actuated into an open state so that at least a portion of the water in the circulation loop can be delivered to the boiler 4.

Although not illustrated in Fig. 1 the recirculation passage 28 may in at least some exemplary embodiments be provided with a second valve in order to prevent recirculation, for example, when water is fed to the boiler 4. In other exemplary embodiments, however, the recirculation passage 28 may always be open, thus allowing some amount of water to be recirculated even when water is passed to the boiler 4. In such case, the recirculation passage 28 may suitably be provided with a flow reducing orifice, so that the flow to the boiler 4 is larger than the flow back to the container 2.

When executing the method steps disclosed herein, the control unit 40 may suitably execute the step (S2) of recirculating the water through the degassing filter 20 in such way that the water is recirculated through the degassing filter 20 so that the total volume that is passed through the degassing filter 20 before said at least a portion of the water is passed to the boiler 4 is at least double the volume of water that was initially present in the container 2 or in the circulation loop before starting to pump water from the container 2.

In particular, the control unit 40 may continue to control the recirculation of the water through the degassing filter 20, keeping the first valve 26 closed and continuing to operate the pump 12 so that the water circulates in the circulation loop, until a satisfactory degassing has been achieved. The control unit 40 may, for instance, continue the recirculation until it determines that the water has been circulated through the circulation loop, and thus through the degassing filter 20, for at least a minimum time period, T1. Such a minimum time period T1 may, for instance, be available from a look-up table or may be calculated by the control unit 40, for example based on the volume of water available in the circulation loop. A larger water volume may require a longer time period T1 than a smaller water volume to arrive at the desired low or zero NCG content in the water. Instead of, or as a supplement to, such a time-based recirculation control, the control unit 40 may instead execute a volume-based recirculation control. In the latter case, the control unit 40 controls the circulation of the water (operating the pump 12 and closing the first valve 26) until at least a minimum number of cycles through the degassing filter 20 has been completed, i.e. the volume of water present in the circulation loop should have passed the degassing filter 20 a minimum number of times. For this purpose, there may suitably be provided a flow meter in the circulation loop.

In other exemplary embodiments, the control unit 40 may as an alternative or as a supplement, perform a pressure-based control of the recirculation. As illustrated in Fig. 1, a pressure sensor 42 may be provided to monitor pressure, for example in the vacuum tank 22. By monitoring the pressure rate of change, the control unit 40 may determine when the recirculating water has become sufficiently degassed, as discussed previously in this disclosure. Fig. 1 also illustrates that the steam sterilizer system 1 may, in at least some exemplary embodiments, be provided with a Total Dissolved Gas (TDG) sensor 44, for example located in the discharge passage 18 between the pump 12 and the degassing filter 20. The control unit 40 may then control the duration of the recirculation based on the NCG content measured by the TDG sensor 44.

In addition to controlling that the duration of the recirculation is sufficient for adequate degassing, the control unit 40 may suitably also receive input from level sensors or level switches 10 of the boiler for determining that the liquid water level in the boiler 4 has sunk to or below a predefined water level, and that a new batch of water may be filled into the boiler 4 for steam generation. Thus, the control unit 40 may monitor the liquid water level in the boiler 4 and proceed from the step (S2) of recirculating the water through the degassing filter 20 to the step (S3) of passing at least a portion of the water to the boiler 4 when the liquid water level if the boiler 4 has reached or fallen below said predefined water level.

The control unit 40 may also perform control actions based on predictions. For instance, the control unit may, based on various input signals, including signals indicative of the water level in the boiler 4, predict that the water level in the boiler 4 will within a predetermined time period T2 fall to or below said predefined water level. In such case, the control unit 40 may, for instance, close the inlet 14 of the container 2, or close a further valve 46 at the inlet 14 of the container 2, in order to prevent fresh water (carrying high NCGs) from being supplied to the container 2 until said predefined water level in the boiler 4 has been reached and degassed water from the container 2 has been passed to the boiler 4 to refill the boiler 4. When the boiler 4 has been refilled, the control unit 40 may again enable fresh water to be supplied into the container 2. As previously explained in this disclosure, in other exemplary embodiments, the fresh water may be supplied to the container 2 in other ways as well, for example upstream or downstream of the container 2 somewhere along the circulation loop. In such cases, the entry of fresh water into the circulation loop may similarly be closed to prevent fresh water from being supplied to the container 2.

As should be understood from the above discussion, the control unit 40 may be configured to prevent fresh water (e.g. by closing the further valve 46) from being supplied to the container 2 while water is recirculated through the degassing filter 20, and to subsequently pass at least a portion of said water to the boiler 4, and subsequently supplying fresh water to the container 2.

In addition to receiving input about the water level in the boiler 4, the control unit 40 may also receive input (e.g. from one or more level sensors or level switches 48) about water level in the container 2, and may determine whether or not it is appropriate to supply fresh water to the container 2. In other words, the control unit 40 may monitor the water level in the container 2 and control the supply of fresh water to the container 2 based on the current water level in the container 2. Typically, when the current water level in the container 2 has fallen to or below a predefined water level, the control unit 40 may decide to supply fresh water to the container 2. Naturally, the control unit 40 may take other considerations into account as well, before deciding on supplying fresh water to the container 2, such as if water is currently being fed to the boiler 4, or expected to be fed to the boiler within a certain (short) time period, in which case the risk of fresh water having a high NCG content being blended with the degassed water already present in the circulation loop should suitably be avoided.

If the control unit 40 predicts that refilling the boiler 4 will not be required soon, then fresh water may suitably be provided to the container 2. Conversely, if the control unit 40 determines that within a certain (short) period of time water will be required to be provided to the boiler 4, then the control unit 40 may prevent the provision of fresh water to the container 2.

It should be understood that after fresh water has been supplied to the container 2, the water in the container 2 needs to be circulated and subjected to degassing. Therefore, the control unit 40 is configured to reiterate the pumping and recirculation of water through the degassing filter 20 while preventing water from flowing from the degassing filter 20 to the boiler 4 (i.e. keeping the first valve 24 closed while operating the pump 12 in the example shown in Fig. 1).

Suitably, if the control unit 40 determines that after filling the boiler 4 with water, there is still sufficient remaining water in the container 2 (or in the circulation loop) for a subsequent batch when water will again be required to the boiler 4, then the control unit 40 may suitably postpone supplying fresh water to the container 2. For instance, the control unit 40 may be configured to monitor the water level in the container 2, and prevent providing fresh water to the container 2 as long as the water level in the container 2 is above or equal to a predefined water level (indicative of a sufficient volume of water remaining in the container for at least another batch of water to be delivered to the boiler 4). Subsequently to operating the boiler 4 to generate steam (and as long as the water level in the container 2 is above or equal to said predefined water level), the control unit 40 may control a further portion or portions of the water to be passed to the boiler 4 and operate the boiler 4 to turn said further portion or portions of the water into steam.

It should be understood that, normally, degassing will be more time-consuming then filling the container 2 with fresh water. Therefore, the control unit 40 may be programed to optimize the filling and/or the degassing based on the current needs and requirements from for example the boiler 4. For instance, the control unit 40 may determine to only fill the container 2 halfway. Degassing half the water volume is expected to take approximately half the time compare to degassing the otherwise full water volume. This may be a strategy if the control unit 40 determines that the boiler 4 will shortly need refilling.

From the above, it should thus be understood that the general approach of recirculating the water through a degassing filter 20 (that is, passing the water through the degassing filter, on average, two or more times) before allowing at least a portion of the water to be delivered to a boiler (e.g. boiler 4 in Fig. 1) will result in a lower amount of NCGs in the steam provided to a steam sterilizer (e.g. sterilizer 1 in Fig. 1), thereby enabling an improved sterilization of articles such as medical goods. In addition to this concept of recirculating water through a degassing filter, other control actions may be included to further reduce the risk of an undesirable NCG content following the steam to the steam sterilizer. For instance, before filling the boiler with water, or at start-up, vacuum-puling in the boiler may be performed in order to remove air in the boiler to prevent air residuals to follow the steam to the steam sterilizer. Such vacuum-pulling is schematically indicated by the arrow above the boiler 4 in Fig. 1.

Fig. 3 is a schematic diagram of a steam sterilizer 60 which could be used with the disclosed system 1 of Fig. 1. Thus, the steam sterilizer 60 in Fig . 3 could suitably take the place of the generally indicated steam sterilizer 6 in Fig. 1. However, other steam sterilizers are also conceivable.

The steam sterilizer 60 comprises a sterilization chamber 62. Steam from the boiler 4 is passed through an inlet 64 into the sterilization chamber 62. A drain system 66 is provided for controlling the discharge of fluid from the sterilization chamber 62 via an outlet 68. A vacuum system 70 (which may be a separate vacuum system or shared with the degassing arrangement in Fig. 1) is connected to the drain system 66 for evacuating the steam from the sterilization chamber 62 and condensing the evacuated steam using a flow of coolant 72. The control unit 40 may be configured to control the flow of steam from the boiler 4 to the sterilization chamber 62 via a controllable valve 74. The control unit 40 may use information received from a temperature sensor 76 in the flow of coolant 72 to determine the amount of steam/condensate admitted from the sterilization chamber 62 to the drain system 66. The control unit 40 may receive other input signals than temperature signals. For instance, the control unit 40 may monitor the pressure inside the sterilization chamber 62 by means of a pressure sensor 78 configured to detect the pressure inside the sterilization chamber 62.

This disclosure includes steam sterilizing systems, in particular for sterilizing articles for medical use. This disclosure also includes controllers (comprising electronics and electronic instructions) for controlling components of the steam sterilizing system as described herein. This disclosure further includes methods of operating and controlling steam sterilizing systems, methods of efficiently degassing water before providing the water to a boiler for steam generation, and methods of sterilizing medical goods. It should be understood that various features and methods disclosed herein are contemplated and disclosed in their various combinations and sub-combinations.

## Claims

1. A method of controlling a steam sterilizer system (1) comprising a boiler (4) in which liquid water is turned into steam, the method comprising:
- pumping water from a container (2) and passing the pumped water through a degassing filter (20) located upstream of the boiler (4) in order to remove non-condensable gases (NCGs) dissolved in the water,
- subsequently to passing the water through the degassing filter (20), recirculating the water through the degassing filter (20) for additional removal of NCGs, while preventing water from flowing from the degassing filter (20) to the boiler (4), and
- subsequently to recirculating the water through the degassing filter (20), passing at least a portion of the water to the boiler (4).

2. The method as claimed in claim 1, wherein said step of recirculating the water through the degassing filter (20) comprises:
- recirculating water through the degassing filter (20) so that the total volume that is passed through the degassing filter (20) before said at least a portion of the water is passed to the boiler i (4) is at least double the volume of water that was initially present in the container (2) before performing said step of pumping water from the container (2).

3. The method as claimed in any preceding claim, wherein the step of recirculating the water through the degassing filter (20) is continued until determining that either (i) the water has been circulated through the degassing filter (20) for at least a minimum time period, T1. or (ii) the water has completed at least a minimum number of cycles through the degassing filter (20).

4. The method as claimed in any preceding claim, comprising:
- monitoring the liquid water level in the boiler (4),
- proceeding from said step of recirculating the water through the degassing filter (20) to said step of passing at least a portion of the water to the boiler (4) when the liquid water level in the boiler (4) has reached or fallen below a predefined water level.

5. The method as claimed in claim 4, comprising:
- determining that the water level in the boiler (4) will within a determined time period, T2, reach or fall below said predefined water level, and
- preventing fresh water from being supplied to the container (2) until said predefined water level in the boiler (4) has been reached and degassed water from the container (2) has been passed to the boiler (4) to refill the boiler (4),
- subsequently to said step of preventing fresh water from being supplied, supplying fresh water to the container (2).

6. The method as claimed in any preceding claim, further comprising:
- preventing fresh water from being supplied to the container (2) while water is recirculated through the degassing filter (20), and subsequently
- passing at least a portion of said water to the boiler (4), and subsequently
- supplying fresh water to the container (2).

7. The method as claimed in any one of the preceding claims, comprising:
- monitoring the water level in the container (2), and
- controlling the supply of fresh water to the container (2) based on the current water level in the container (2).

8. The method as claimed in claim 7, wherein said step of controlling the supply of fresh water to the container (2) based on the current water level in the container (2) comprises:
- supplying fresh water to the container (2) when the water level is below a predefined level.

9. The method of any of the preceding claims, further comprising at least one of:
- providing fresh water to the container (2) in response to a determination that degassed water will not be provided to the boiler (4) for at least a first time period, T3; and
- preventing providing fresh water to the container (2) in response to a determination that water will need to be provided to the boiler (4) within a second time period, T4.

10. The method as claimed in any preceding claim, comprising:
- subsequently to said step of passing at least a portion of the water to the boiler (10), preventing water from passing to the boiler (4), and
- supplying fresh water to the container (2), and then
- reiterating said steps of pumping and recirculating water through the degassing filter (20) while preventing water from flowing from the degassing filter (20) to the boiler (4).

11. The method as claimed in any one of claims 1-10, comprising:
- subsequently to said step of passing at least a portion of the water to the boiler (4), operating the boiler (4) to generate steam, and
- monitoring the water level in the container (2), and
- preventing providing fresh water to the container (2) as long as the water level in the container (2) is above or equal to a predefined water level, and
- subsequently to said step of operating the boiler (4) to generate steam, and as long as the water level in the container (2) is above or equal to said predefined water level, passing a further portion or portions of the water to the boiler (4) and operate the boiler (4) to generate steam.

12. The method as claimed in any preceding claim, comprising:
- applying, by means of a vacuum tank (22), a vacuum to the degassing filter (20) to draw NCGs from water passing through the degassing filter (20).

13. The method as claimed in any one of claims 1-12, wherein the step of recirculating the water through the degassing filter (20) is continued until determining that there is a pressure rate of change below a predefined threshold in a closable volume in gaseous communication with the degassing filter (20).

14. A control unit configured to carry out the steps of the method according to any preceding claim, the control unit being configured to:
- control a pump (12) of the steam sterilizer system (1), thereby controlling the pumping of water from the container (2),
- controlling a first valve (26) of the steam sterilizer system (1) to selectively prevent or enable water to be passed from the degassing filter (20) to the boiler (4), and
- optionally, controlling a further valve (46) of the steam sterilizer system (1) to selectively prevent or enable fresh water to be supplied to the container (2).

15. A steam sterilizer system (1) comprising the control unit of claim 14, and further comprising a boiler (4) and a sterilization chamber (6).

## Patentansprüche

1. Verfahren zum Steuern eines Dampfsterilisierungssystems (1), das einen Kessel (4) umfasst, in dem flüssiges Wasser in Dampf umgewandelt wird, wobei das Verfahren Folgendes umfasst:
- Pumpen von Wasser aus einem Behälter (2) und Leiten des gepumpten Wassers durch einen Entgasungsfilter (20), der sich dem Kessel (4) vorgelagert befindet, um nicht kondensierbare Gase (NCGs), die in dem Wasser gelöst sind, zu entfernen,
- nach dem Leiten des Wassers durch den Entgasungsfilter (20), Rezirkulieren des Wassers durch den Entgasungsfilter (20) zur zusätzlichen Entfernung von NCGs, während verhindert wird, dass Wasser von dem Entgasungsfilter (20) in den Kessel (4) fließt, und
- nach dem Rezirkulieren des Wassers durch den Entgasungsfilter (20), Leiten von mindestens einem Teil des Wassers in den Kessel (4).

2. Verfahren nach Anspruch 1, wobei der Schritt des Rezirkulierens des Wassers durch den Entgasungsfilter (20) Folgendes umfasst:
- Rezirkulieren des Wassers durch den Entgasungsfilter (20), so dass das Gesamtvolumen, das durch den Entgasungsfilter (20) geleitet wird, bevor der mindestens eine Teil des Wassers in den Kessel (4) geleitet wird, mindestens das Doppelte des Wasservolumens beträgt, das ursprünglich in dem Behälter (2) vorhanden war, bevor der Schritt des Pumpens von Wasser aus dem Behälter (2) durchgeführt wurde.

3. Verfahren nach einem der vorhergehenden Ansprüche, wobei der Schritt des Rezirkulierens des Wassers durch den Entgasungsfilter (20) fortgesetzt wird, bis bestimmt wird, dass entweder (i) das Wasser für mindestens einen Mindestzeitraum, T1, durch den Entgasungsfilter (20) zirkuliert wurde oder (ii) das Wasser mindestens eine Mindestanzahl von Zyklen durch den Entgasungsfilter (20) absolviert hat.

4. Verfahren nach einem der vorhergehenden Ansprüche, umfassend:
- Überwachen des Pegels des flüssigen Wassers in dem Kessel (4),
- Übergehen von dem Schritt des Rezirkulierens des Wassers durch den Entgasungsfilter (20) zu dem Schritt des Leitens von mindestens einem Teil des Wassers zu dem Kessel (4), wenn der Pegel des flüssigen Wassers in dem Kessel (4) einen vordefinierten Wasserpegel erreicht hat oder unter diesen gefallen ist.

5. Verfahren nach Anspruch 4, umfassend:
- Bestimmen, dass der Wasserpegel in dem Kessel (4) innerhalb eines bestimmten Zeitraums, T2, den vordefinierten Wasserpegel erreichen oder unterschreiten wird, und
- Verhindern, dass frisches Wasser zu dem Behälter (2) zugeführt wird, bis der vordefinierte Wasserpegel in dem Kessel (4) erreicht ist und entgastes Wasser aus dem Behälter (2) in den Kessel (4) geleitet wurde, um den Kessel (4) aufzufüllen,
- nach dem Schritt des Verhinderns, dass frisches Wasser zugeführt wird, Zuführen von frischem Wasser zu dem Behälter (2).

6. Verfahren nach einem der vorhergehenden Ansprüche, ferner umfassend:
- Verhindern, dass frisches Wasser zu dem Behälter (2) zugeführt wird, während Wasser durch den Entgasungsfilter (20) rezirkuliert wird, und danach
- Leiten von mindestens einem Teil des Wassers zu dem Kessel (4) und danach
- Zuführen von frischem Wasser zu dem Behälter (2).

7. Verfahren nach einem der vorhergehenden Ansprüche, umfassend:
- Überwachen des Wasserpegels in dem Behälter (2) und
- Steuern der Zufuhr von frischem Wasser zu dem Behälter (2) basierend auf dem aktuellen Wasserpegel in dem Behälter (2).

8. Verfahren nach Anspruch 7, wobei der Schritt des Steuerns der Zufuhr von frischem Wasser zu dem Behälter (2) basierend auf dem aktuellen Wasserpegel in dem Behälter (2) Folgendes umfasst:
- Zuführen von frischem Wasser zu dem Behälter (2), wenn der Wasserpegel unterhalb eines vordefinierten Pegels liegt.

9. Verfahren nach einem der vorhergehenden Ansprüche, ferner umfassend mindestens eines des Folgenden:
- Bereitstellen von frischem Wasser an den Behälter (2) als Reaktion auf eine Bestimmung, dass dem Kessel (4) für mindestens einen ersten Zeitraum, T3, kein entgastes Wasser bereitgestellt werden wird; und
- Verhindern eines Bereitstellens von frischem Wasser an den Behälter (2) als Reaktion auf eine Bestimmung, dass dem Kessel (4) innerhalb eines zweiten Zeitraums, T4, Wasser bereitgestellt werden muss.

10. Verfahren nach einem der vorhergehenden Ansprüche, umfassend:
- nach dem Schritt des Leitens von mindestens einem Teil des Wassers in den Kessel (10), Verhindern, dass Wasser in den Kessel (4) geleitet wird, und
- Zuführen von frischem Wasser zu dem Behälter (2) und dann
- Wiederholen der Schritte des Pumpens und Rezirkulierens von Wasser durch den Entgasungsfilter (20), während gleichzeitig verhindert wird, dass Wasser von dem Entgasungsfilter (20) in den Kessel (4) fließt.

11. Verfahren nach einem der Ansprüche 1-10, umfassend:
- nach dem Schritt des Leitens von mindestens einem Teil des Wassers in den Kessel (4), Betreiben des Kessels (4), um Dampf zu erzeugen, und
- Überwachen des Wasserpegels in dem Behälter (2) und
- Verhindern, dass dem Behälter (2) frisches Wasser bereitgestellt wird, solange der Wasserpegel in dem Behälter (2) über einem vordefinierten Wasserpegel liegt oder gleich diesem ist, und
- nach dem Schritt des Betreibens des Kessels (4), um Dampf zu erzeugen, und solange der Wasserpegel in dem Behälter (2) über dem vordefinierten Wasserpegel liegt oder gleich diesem ist, Leiten eines weiteren Teils oder weiterer Teile des Wassers zu dem Kessel (4) und Betreiben des Kessels (4), um Dampf zu erzeugen.

12. Verfahren nach einem der vorhergehenden Ansprüche, umfassend:
- Anlegen, mittels eines Vakuumtanks (22), eines Vakuums an den Entgasungsfilter (20), um NCGs aus dem Wasser, das durch den Entgasungsfilter (20) fließt, zu ziehen.

13. Verfahren nach einem der Ansprüche 1-12, wobei der Schritt des Rezirkulierens des Wassers durch den Entgasungsfilter (20) fortgesetzt wird, bis bestimmt wird, dass in einem verschließbaren Volumen in Gasverbindung mit dem Entgasungsfilter (20) eine Druckänderungsrate unterhalb eines vordefinierten Schwellenwerts vorliegt.

14. Steuereinheit, die dazu konfiguriert ist, die Schritte des Verfahrens nach einem der vorhergehenden Ansprüche umzusetzen, wobei die Steuereinheit zu Folgendem konfiguriert ist:
- Steuern einer Pumpe (12) des Dampfsterilisierungssystems (1) und dadurch Steuern des Pumpens von Wasser aus dem Behälter (2),
- Steuern eines ersten Ventils (26) des Dampfsterilisierungssystems (1), um selektiv zu verhindern oder zu ermöglichen, dass Wasser von dem Entgasungsfilter (20) zu dem Kessel (4) geleitet wird, und
- optional, Steuern eines weiteren Ventils (46) des Dampfsterilisierungssystems (1), um selektiv zu verhindern oder zu ermöglichen, dass frisches Wasser zu dem Behälter (2) zugeführt wird.

15. Dampfsterilisierungssystem (1), das die Steuereinheit nach Anspruch 14 umfasst und ferner einen Kessel (4) und eine Sterilisierungskammer (6) umfasst.

## Revendications

1. Procédé de commande d'un système de stérilisateur à la vapeur (1) comprenant une chaudière (4) dans laquelle de l'eau liquide est transformée en vapeur, le procédé comprenant :
- le pompage d'eau depuis un récipient (2) et le passage de l'eau pompée à travers un filtre de dégazage (20) situé en amont de la chaudière (4) afin d'éliminer les gaz non condensables (GNC) dissous dans l'eau,
- après le passage de l'eau à travers le filtre de dégazage (20), la recirculation de l'eau à travers le filtre de dégazage (20) pour éliminer davantage de NCG, tout en empêchant l'eau de s'écouler du filtre de dégazage (20) vers la chaudière (4), et
- ensuite la recirculation de l'eau à travers le filtre de dégazage (20), en faisant passer au moins une partie de l'eau vers la chaudière (4).

2. Procédé selon la revendication 1, dans lequel ladite étape de recirculation de l'eau à travers le filtre de dégazage (20) comprend :
- la recirculation de l'eau à travers le filtre de dégazage (20) de sorte que le volume total qui passe à travers le filtre de dégazage (20) avant que ladite au moins une partie de l'eau ne passe à la chaudière (4) soit au moins le double du volume d'eau qui était initialement présent dans le récipient (2) avant d'effectuer ladite étape de pompage de l'eau du récipient (2).

3. Procédé selon l'une quelconque des revendications précédentes, dans lequel l'étape de recirculation de l'eau à travers le filtre de dégazage (20) est poursuivie jusqu'à ce qu'il soit déterminé que (i) l'eau a circulé à travers le filtre de dégazage (20) pendant au moins une période de temps minimale, T1 ou (ii) l'eau a effectué au moins un nombre minimum de cycles à travers le filtre de dégazage (20).

4. Procédé selon l'une quelconque des revendications précédentes, comprenant :
- la surveillance du niveau d'eau liquide dans la chaudière (4),
- le passage de l'étape de recirculation de l'eau à travers le filtre de dégazage (20) à l'étape de passage d'au moins une partie de l'eau vers la chaudière (4) lorsque le niveau d'eau liquide dans la chaudière (4) a atteint ou est descendu en dessous d'un niveau d'eau prédéfini.

5. Procédé selon la revendication 4, comprenant :
- la détermination que le niveau d'eau dans la chaudière (4) atteindra ou descendra en dessous dudit niveau d'eau prédéfini dans un laps de temps déterminé, T2, et
- l'empêchement de l'alimentation en eau fraîche du récipient (2) jusqu'à ce que le niveau d'eau prédéfini dans la chaudière (4) soit atteint et que l'eau dégazée du récipient (2) soit passée à la chaudière (4) pour remplir la chaudière (4),
- suite à ladite étape consistant à empêcher l'alimentation en eau fraiche, alimenter en eau fraiche le récipient (2).

6. Procédé selon l'une quelconque des revendications précédentes, comprenant en outre :
- l'empêchement de l'alimentation en eau fraîche du récipient (2) pendant que l'eau est recirculée à travers le filtre de dégazage (20), et ensuite
- le passage d'au moins une partie de ladite eau vers la chaudière (4), ensuite
- l'alimentation en eau fraiche du récipient (2).

7. Procédé selon l'une quelconque des revendications précédentes, comprenant :
- la surveillance du niveau d'eau dans le récipient (2), et
- la commande de l'alimentation en eau fraiche du récipient (2) en fonction du niveau d'eau actuel dans le récipient (2).

8. Procédé selon la revendication 7, dans lequel ladite étape de commande de l'alimentation en eau fraiche du récipient (2) en fonction du niveau d'eau actuel dans le récipient (2) comprend :
- l'alimentation en eau fraiche du récipient (2) lorsque le niveau d'eau est inférieur à un niveau prédéfini.

9. Procédé selon l'une quelconque des revendications précédentes, comprenant en outre au moins l'un des éléments suivants :
- l'alimentation en eau fraîche du récipient (2) en réponse à une détermination selon laquelle de l'eau dégazée ne sera pas fournie à la chaudière (4) pendant au moins une première période de temps, T3 ; et
- l'empêchement de l'alimentation en eau fraîche du récipient (2) en réponse à une détermination selon laquelle de l'eau devra être fournie à la chaudière (4) dans une deuxième période de temps, T4.

10. Procédé selon l'une quelconque des revendications précédentes, comprenant :
- suite à ladite étape de passage d'au moins une partie de l'eau vers la chaudière (10), l'empêchement de l'eau de passer vers la chaudière (4), et
- l'alimentation en eau fraiche du récipient (2), puis
- la réitération desdites étapes de pompage et de recirculation de l'eau à travers le filtre de dégazage (20) tout en empêchant l'eau de s'écouler du filtre de dégazage (20) vers la chaudière (4).

11. Procédé selon l'une quelconque des revendications 1 à 10, comprenant :
- après ladite étape de passage d'au moins une partie de l'eau vers la chaudière (4), le fonctionnement de la chaudière (4) pour générer de la vapeur, et
- la surveillance du niveau d'eau dans le récipient (2), et
- l'empêchement de l'alimentation en eau fraiche du récipient (2) tant que le niveau d'eau dans le récipient (2) est supérieur ou égal à un niveau d'eau prédéfini, et
- suite à ladite étape de fonctionnement de la chaudière (4) pour générer de la vapeur, et tant que le niveau d'eau dans le récipient (2) est supérieur ou égal audit niveau d'eau prédéfini, le passage d'une ou plusieurs autres parties de l'eau à la chaudière (4) et le fonctionnement de la chaudière (4) pour générer de la vapeur.

12. Procédé selon l'une quelconque des revendications précédentes, comprenant :
- l'application, au moyen d'un réservoir à vide (22), d'un vide au filtre de dégazage (20) pour extraire les NCG de l'eau traversant le filtre de dégazage (20).

13. Procédé selon l'une quelconque des revendications 1 à 12, dans lequel l'étape de recirculation de l'eau à travers le filtre de dégazage (20) est poursuivie jusqu'à ce qu'il soit déterminé qu'il existe un taux de changement de pression inférieur à un seuil prédéfini dans un volume fermable en communication gazeuse avec le filtre de dégazage (20).

14. Unité de commande configurée pour mettre en œuvre les étapes du procédé selon l'une quelconque des revendications précédentes, l'unité de commande étant configurée pour :
- commander une pompe (12) du système de stérilisateur à la vapeur (1), commandant ainsi le pompage de l'eau du récipient (2),
- commander une première vanne (26) du système de stérilisateur à la vapeur (1) pour empêcher ou permettre sélectivement le passage de l'eau du filtre de dégazage (20) vers la chaudière (4), et
- éventuellement, commander une autre vanne (46) du système de stérilisateur à la vapeur (1) pour empêcher ou permettre sélectivement l'alimentation en eau fraîche du récipient (2).

15. Système de stérilisateur à la vapeur (1) comprenant l'unité de commande selon la revendication 14, et comprenant en outre une chaudière (4) et une chambre de stérilisation (6).
